(19) Europäisches Patentamt
European Patent Office
Office européen des brevets

(11) Numéro de publication: **0 377 370 A1**

(12) **DEMANDE DE BREVET EUROPEEN**

(21) Numéro de dépôt: **89403546.8**

(22) Date de dépôt: **19.12.89**

(51) Int. Cl.5: **B01J 13/04, A61K 9/50, A01N 25/24**

(30) Priorité: **22.12.88 FR 8817274**

(43) Date de publication de la demande: **11.07.90 Bulletin 90/28**

(84) Etats contractants désignés: **AT BE CH DE ES FR GB GR IT LI LU NL SE**

(71) Demandeur: **RHONE-POULENC SANTE**
**20, avenue Raymond Aron**
**F-92160 Antony(FR)**

(72) Inventeur: **Prud'homme, Christian**
**19 rue Commandant Faurax**
**F-69006 Lyon(FR)**
Inventeur: **Porte, Hugues**
**6 chemin de Crépieux**
**F-63900 Caluire(FR)**

(74) Mandataire: **Le Pennec, Magali et al**
**RHONE-POULENC SANTE, Service Brevets,**
**20 Avenue Raymond Aron**
**F-92165 Antony Cédex(FR)**

(54) **Procédé d'encapsulation de particules au moyen d'un copolymère silicone thermoplastique et particules enrobées.**

(57) La présente invention concerne un procédé d'encapsulation de particules de principe actif par pulvérisation d'un copolymère silicone thermoplastique en solution dans un solvant organique ou en dispersion ou en émulsion aqueuse et élimination par séchage à l'air chaud du solvant ou de l'eau. Le procédé de pulvérisation/séchage utilisé est par exemple le procédé WURSTER.

Le copolymère utilisable est formé d'une succession de segments ou blocs polydiorganosiloxane et de segments ou blocs organiques (polyesters, polyethers, polyuréthannes, polystyrènes, etc ...). Il peut être également un polydiorganosiloxane greffé par des chaînes organiques. Le principe actif se présente sous forme de particules de granulométrie comprise entre 50 µm et 5 mm. Le principe actif peut être un catalyseur, un parfum, un colorant, un produit cosmétique, un médicament, un produit phytosanitaire, des semences végétales, etc ...

Application notamment à la liberation contrôlée d'un principe actif en milieu aqueux.

EP 0 377 370 A1

# PROCEDE D'ENCAPSULATION DE PARTICULES AU MOYEN D'UN COPOLYMERE SILICONE THERMOPLASTIQUE ET PARTICULES ENROBEES

L'encapsulation de principes actifs se présentant sous forme de particules dont la granulométrie varie de quelques µm à quelques millimètres a pris une importance tout à fait considérable principalement (mais pas uniquement) dans le domaine pharmaceutique et phytosanitaire.

Cette encapsulation a pour but la protection, puis la libération contrôlée du principe actif notamment dans un milieu aqueux.

Les polymères silicones sont utilisés depuis longtemps comme matière constitutive du revêtement encapsulant le principe actif, les polymères silicones présentent deux propriétés précieuses dans ce type d'application, à savoir leur bio-compatibilité et leur perméabilité aux gaz et aux petites molécules.

Les silicones sont généralement utilisés sous forme de macromolécules porteuses de fonctions chimiques réactives en vue de leur réticulation à la surface du principe actif pour constituer le revêtement formé de polymères réticulés insolubles dans les solvants organiques usuels.

Les brevets EP-A-267 003, EP-A-280 400 et US-A-4 370 160 sont des illustrations de cet état de la technique.

Selon cette technique, la réticulation de ces polymères silicones se fait en présence du principe actif. Cette réticulation se fait généralement au moyen d'un catalyseur organométallique de durcissement, d'un chauffage, ou d'un rayonnement (ultra-violet, infra-rouge, faisceau d'électrons, gamma), ou par une combinaison de ces moyens. Cette réticulation faisant intervenir des réactions chimiqes ou photochimiques en présence du principe actif, peut donc avoir une action néfaste sur ce principe actif.

Par ailleurs, les propriétés physicochimiques intrinsèques du principe actif peuvent perturber, voir inhiber complètement le processus de réticulation, ce problème est particulièrement important lorsque le principe actif comporte une fonction thiol.

En outre dans les applications pharmaceutiques et biologiques, le caractère biocompatible du polymère a une importance fondamentale. Le polymère servant à encapsuler un principe actif peut difficilement être débarrassé des produits indésirables tels que catalyseurs, polymères résiduels non intégrés au réticulat.

A l'heure actuelle le procédé d'encapsulation par pelliculage successif le plus performant sur le plan technique pour réaliser l'encapsulation de principes actifs est le procédé comprenant au moins une étape de pulvérisation d'une composition pelliculante contenant un polymère organique en solution dans un solvant organique ou sous la forme d'une émulsion ou d'une dispersion aqueuse suivie d'au moins un séchage, c'est-à-dire de l'évaporation du solvant organique et/ou de l'eau.

Un exemple d'un tel procédé est le procédé connu sous l'appellation "spray coating" selon lequel les particules à encapsuler sont brassées (fluidisées) par un flux gazeux qui assure également le séchage, c'est-à-dire l'évaporation du solvant organique et/ou de l'eau.

La composition pelliculante est pulvérisée par une ou plusieurs buses situées à différentes zones du réacteur suivant le type de procédé utilisé par exemple, au-dessus, à l'intérieur ou à la base du nuage de particules.

Ainsi cette pulvérisation est à la base du lit fluidisé de particules dans le procédé WURSTER. La technique de pulvérisation de WURSTER est décrite en détails dans les brevets US-A-2 799 241, US-A-3 089 824, US-A-3 117 027, US-A-3 196 827, US-A-3 207 824, US-A-3 241 520, US-A-3 253 994 et EP-A-188 953.

Dans le but d'utiliser les propriétés physico-chimiques précieuses des silicones dans le cadre de la technique du procédé de pulvérisation/séchage sans avoir les inconvénients des réticulats, l'homme de métier peut être enclin à penser d'utiliser les polymères diorganopolysiloxanes huiles ou gommes non réticulables, c'est-à-dire non réactives dans un procédé de pelliculage par pulvérisation/séchage de particules de principes actifs.

Malheureusement, comme il est démontré dans les exemples comparatifs de la présente demande, on montre que ces huiles ou gommes silicones non réactives sont particulièrement inadaptées pour la mise en oeuvre du procédé d'encapsulation par pulvérisation/séchage.

En effet, la particule encapsulée présente au cours du procédé d'encapsulation et à fortiori, en fin de procédé, une tendance marquée à l'agglomération due au caractère collant et poisseux du polymère silicone. En outre, même en l'absence de caractère collant, le revêtement silicone a tendance à fluer et la particule n'est plus parfaitement encapsulée.

Le but visé par la présente invention est précisément de proposer une classe de polymères utilisable dans un procédé d'encapsulation de particules de principe actif, par pelliculage au moyen d'un procédé de pulvérisation/séchage qui présente les mêmes avantages que les polymères silicones mais sans présenter

les inconvénients des capsules de réticulats des polymères silicones et les inconvénients des capsules de polymères silicones non réactifs.

Ce but est atteint par la présente invention qui concerne en effet un procédé d'encapsulation de particules de principe actif de granulométrie moyenne comprise entre 50 µm et 5 mm, par pelliculage de chaque particule au moyen d'un polymère, la pellicule étant obtenue par pulvérisation sur chaque particule d'un polymère en solution dans un solvant organique ou en émulsion ou dispersion aqueuse, suivie de l'évaporation du solvant et/ou de l'eau par séchage, caractérisé en ce que le polymère utilisé est un copolymère silicone thermoplastique.

Les copolymères silicones thermoplastiques utilisables dans le cadre de la présente invention sont choisis parmi les copolymères multi-séquencés linéaires, les copolymères à blocs et les copolymères greffés dont la chaîne polymère principale sensiblement linéaire est constituée soit par une alternance de segments ou blocs polydiorganosiloxanes et de segments ou blocs organiques, soit par une chaîne polydiorganosiloxane sur laquelle sont greffés des chaînes organiques.

Les radicaux organiques des motifs diorganosiloxyle sont de préférence des radicaux alkyle en $C_1$-$C_4$, en particulier méthyle, des radicaux trifluoro-3,3,3-propyle et des radicaux phényle.

Les copolymères thermoplastiques utilisables dans le cadre de la présente invention sont en outre des polymères qui se ramollissent sous l'effet de la chaleur, qui sont solubles dans certains solvants organiques et/ou émulsifiables ou dispersables dans l'eau selon les techniques usuelles, et dont la mise en forme met en jeu des processus physiques réversibles.

Les copolymères thermoplastiques préférés sont ceux qui présentent une Tg (température de transition vitreuse) ou température de fusion (pour les copolymères semi-cristallins) supérieures à la température ambiante (25 °C) et, de préférence supérieures à 40 °C et généralement inférieures à 200 °C.

Les copolymères silicones thermoplastiques utilisables dans le cadre de la présente invention sont ceux qui sont solubles dans les solvants organiques volatils présentant, de préférence, des vitesses d'évaporation élevées.

Ces solvants, utilisés seuls ou en mélanges, peuvent être en particulier le chloroforme, l'acétone, la méthyléthylcétone, le tétrahydrofurane, le dichloroéthane, le tétrachloroéthane, le tétrachlorure de carbone, le trichloroéthylène, l'hexane, l'heptane, le méthanol, l'éthanol, l'isopropanol et le toluène.

Sont également utilisables, les copolymères silicones thermoplastiques pouvant être mis en dispersion ou en émulsions aqueuses par les techniques usuelles de mise en dispersion ou en émulsion. Pour ce faire il est parfois nécessaire d'ajouter un agent tensio-actif ou un agent dispersant convenable.

Les segments séquencés, blocs ou greffons organiques des copolymères silicones thermoplastiques peuvent être en particulier: - des polyuréthannes (voir par exemple le brevet canadien CA-A- 1 072 241, les brevets américains US-A-4 145 508, US-A-4 180 515 et US-A-4 518 758,
- des polyarylènes (voir par exemple US-A-4 233 427 et FR-A-2 407 950),
- des polystyrènes (voir par exemple US-A-4 263 401),
- des polyesters non dégradables (voir par exemple US-A-3 701 815),
- des polyéthers,
- des polycarbonates (voir par exemple J. Polym. Sci., Polymer Letters, ed. 7, p. 569-577) (1969),
- des polyamides,
- des polyimides,
- des polyimides/amides,

et de façon générale les copolymères silicones thermoplastiques décrits aux pages 181 à 198 de l'édition 1988 de INORGANIC and ORGANOMETALLIC POLYMERS.

Ces segments, blocs ou greffons organiques sont, de préférence, présents suivant une teneur pondérale de 5 à 60 %, de préférence de 8 à 45 en poids par rapport au poids total du copolymère thermoplastique.

Le copolymère est utilisé tel quel ou au sein d'une composition encapsulante où le polymère est en mélange avec d'autres produits améliorant le revêtement (charges, stabilisant U.V., colorant, etc......), ou avec des additifs utiles à l'application visée.

Le principe actif à encapsuler peut être le produit pur ou son association avec un support convenable en vue de lui conférer, entre autres, des propriétés mécaniques convenables et suffisantes pour la mise en oeuvre du procédé d'encapsulation selon l'invention.

Le principe actif que l'on peut encapsuler par le procédé de l'invention peut donc être quelconque à partir du moment où sa granulométrie moyenne est comprise entre 50 µm et 5 mm, et plus particulièrement entre 200 µm et 4 mm.

Ce principe actif peut être de la méthonine, un gel aqueux d'alginate de calcium et de façon plus générale un catalyseur, un colorant, un durcisseur, un détergent, un produit cosmétique, un produit

pharmaceutique, un médicament, une vitamine, un parfum, un enzyme, un aliment, un métal, un agent odorant, un produit phytosanitaire, un pesticide, un fongicide, un insecticide, un herbicide, une phéromone, un substrat solide contenant un constituant actif absorbé, une semence végétale, un embryon végétal, un tissu meristématique, etc

Parmi les principes actifs médicamenteux peuvent être cités :

- les agents antiinflammatoires tels que :
. le kétoprofène,
. l'ibuprofène,
- l'indométhacine,
- les agents hormonaux tels que :
. les stéroïdes,
. les hormones peptidiques,
- les agents antitumoraux,
- les antibactériens tels que :
. les pénicillines,
- les céphalosporines,
- les streptomycines

Le procédé de pulvérisation/séchage utilisé dans le procédé de la présente invention est du type décrit ci-dessus, par exemple, ce peut être le procédé WURSTER décrit en particulier dans les brevets cités ci-dessus et dans l'ouvrage de Agis F. KYDONIEUS "CONTROLLED RELEASE TECHNOLOGIES", Vol. 51 (1980), p. 133.

Selon ce procédé les particules à revêtir (encapsuler) sont brassées (fluidisées) dans un réacteur au moyen d'un courant d'air vertical. Une partie du lit fluidisé peut être délimitée par une partie cylindrique ouverte à ses deux extrémités et parcourue par un courant d'air vertical à haute vitesse créant une circulation des particules.

Le copolymère, en solution dans un solvant organique ou en émulsion ou dispersion aqueuses, est introduit par pulvérisation sous pression à l'intérieur du lit fluidisé de particules et se dépose sur les particules à revêtir tandis que le solvant et/ou l'eau est évaporé par le courant d'air.

Dans d'autres procédés utilisables dans le cadre de la présente invention, le brassage des particules est effectué mécaniquement, par exemple au moyen d'un tambour ou d'un plateau rotatif et le courant gazeux ne sert qu'au séchage.

Pour obtenir une encapsulation efficace, il est recommandé que la pellicule de revêtement présente une épaisseur moyenne comprise entre 1 et 200 µm, de préférence entre 5 et 100 µm.

Bien entendu l'homme de métier, par des essais de routine, peut adapter sans difficulté l'épaisseur du revêtement à la nature du principe actif, à son état de surface, à la nature du copolymère utilisé, à la cinétique désirée de libération du principe actif, etc.....

Il est possible également d'encapsuler les particules par exemple par une première capsule en polymère thermoplastique puis de réaliser une deuxième couche de nature différente et par un procédé différent, par exemple d'une cire naturelle ou synthétique.

Les principes actifs médicamenteux encapsulés par lesdits polymères séquencés organosiloxanes thermoplastiques font aussi partie de l'invention. Ils présentent une cinétique de libération très intéressante lorsqu' ils sont administrés à l'homme.

La plupart des médicaments ont un profil d'activité "in vivo" et pour que ce profil soit atteint demande une formulation pharmaceutique spécifique, particulièrement lorsque l'on recherche une concentration plasmatique soigneusement contrôlée. La disponibilité du médicament sur de longues périodes et la facilité du traitement ambulatoire apportent aussi des exigences en ce qui concerne la nature des excipients de formulation. Chaque malade préfère absorber son médicament une fois par jour plutôt que plusieurs fois sur la même période. En plus, la libération du principe actif doit se faire la plus régulièrement possible de façon, surtout pour les analgésiques, à éviter au malade les crises douloureuses pendant les latences où le principe actif n'est pas actif. Les principes actifs encapsulés de l'invention répondent à cet objectif.

La présente invention sera plus complètement décrite à l'aide de l'exemple suivant qui ne doit pas être considéré comme limitatif de l'invention.

EXEMPLE 1

- Exemple 1.a. : enrobaae de particules de méthionine :

On utilise dans cet exemple un copolymère multiséquencé thermoplastique préparé en suivant le mode opératoire décrit à l'exemple 1 du brevet US-A-4 233 427.

L'α,w-dihydrogénopolydiméthylsiloxane utilisé a une masse moléculaire moyenne : Mn = 8 000g/mole déterminée par dosage des SiH terminaux.

Ce copolymère est formé par une alternance de segments polydiméthylsiloxane et de segments poly{(diméthylsilylène) phénylène-(diméthylsilylène)-1,2-éthanediyl} répondant à la formule générale moyenne :

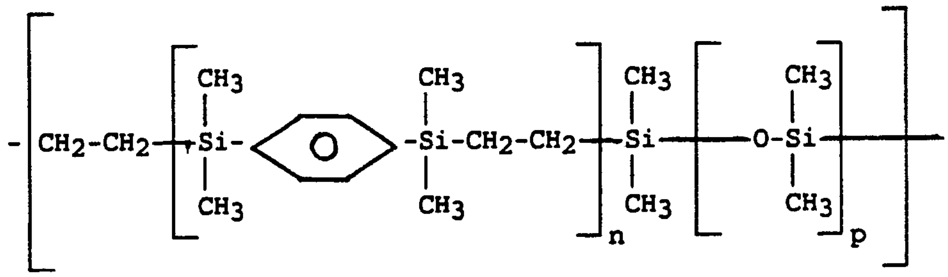

Les caractéristiques physico-chimiques du polymère sont les suivantes :

- viscosité intrinsèque : (20 °C, $CHCl_3$) : 0,77 dl/g
- Mn (masse moléculaire moyenne en nombre) 157 000 g/mole
- Mw (masse moléculaire moyenne en poids) : 305 000 g/mole

Le copolymère contient 75 % en poids de blocs polydiméthylsiloxane.

La masse moléculaire est déterminée par G.P.C. (chromatographie par perméation de gel) à 20 °C dans le tétrahydrofuranne fonctionnant en étalonnage universel.

Ce copolymère se comporte comme un élastomère à l'intérieur d'un domaine de températures allant de -120 °C environ correspondant à la température de transition vitreuse de la partie polydiméthylsiloxane à 170/180 °C correspondant à la température de fusion de la phase rigide semi-cristalline.

On utilise un appareil d'enrobage UNI-GLATT ® équipé d'un système de pulvérisation WURSTER.

On dissout à une température d'environ 55 °C, 40 g de copolymère dans 725 $cm^3$ de dichloro-1,2-éthane PROLABO RECTA PUR ®

On charge dans l'appareil 350 g de granulés de méthionine présentant un titre en acide aminé de 98 % et un diamètre particulaire moyen compris entre 0,8 et 1 mm.

Ces granulés sont mis en suspension dans un flux d'air de fluidisation de 70 $m^3/h$ à 30 °C (température mesurée à la sortie de l'appareil). La solution de copolymère, dont la température est maintenue à 40 °C est pulvérisée au sein du lit fluidisé.

Les conditions de pulvérisation sont les suivantes :

- débit de solution de copolymère : 5 $cm^3/mn$,
- pression de l'air de pulvérisation : 1,5 bar,
- température de l'air de pulvérisation : 30 °C,
- durée de la pulvérisation : 2 heures 40 minutes

On obtient, en fin de pulvérisation, 376 g de particules de méthionine, soit un rendement pondéral de 96,4 %. Les particules enrobées présentent un titre pondéral en méthionine de 89 %.

Le taux d'enrobage obtenu, qui est le rapport du poids du copolymère sur le poids de méthionine enrobée est de 9 %, ce qui correspond à une épaisseur moyenne de la pellicule de copolymère d'environ 15 µm, épaisseur calculée pour une densité du copolymère de 1.

- Exemple 1.b. - libération de méthionine en milieu aqueux des particules de méthionine enrobées :

Dans un réacteur de 2 litres en verre, muni d'un agitateur tournant à 300 tours/minute et thermostaté à 40 °C, on charge:

- 1 litre d'eau déminéralisée,
- 8 grammes de granulés de méthionine enrobée préparés à l'exemple 1.a. ci-dessus,
- 0,2 g de $NaN_3$ (bactéricide)

La libération de méthionine est appréciée en effectuant son dosage sur des prélèvements échelonnés dans le temps de la phase aqueuse.

Les résultats obtenus sont rassemblés dans le tableau I ci-après où T représente le pourcentage de

méthionine libérée et t le temps de séjour (en heure) des granulés dans la phase aqueuse

TABLEAU I

| t (heures) | 2 | 72 | 144 | 240 | 312 | 360 | 408 | 648 |
|---|---|---|---|---|---|---|---|---|
| T (%) | 3 | 12 | 30 | 50 | 60 | 65 | 70 | 100 |

L'essai de libération montre que :
- la quantité de méthionine libérée à une durée t inférieure à 30 minutes est pratiquement nulle, ce qui indique que l'enrobage est de bonne qualité et que le copolymère recouvre bien la totalité de la surface des granules,
- la méthionine est libérée avec une cinétique de libération d'ordre voisin de zéro, ce qui est généralement recherché en pratique dans la plupart des applications,
- l'observation au microscope des particules prélevées après 408 heures d'immersion dans l'eau montre que la méthionine restante dans le granulé se trouve sous forme de solution aqueuse emprisonnée dans la membrane sphérique du copolymère.

- EXEMPLE 2 : Pelliculage de sphères de gel aqueux d'alginate de calcium.

Le copolymère multiséquencé linéaire thermoplastique utilisé dans cet exemple est défini par la même formule générale que le copolymère de l'exemple 1, et est préparé selon le même mode opératoire ; il présente cependant des caractéristiques différentes qui sont les suivantes :
- il est préparé à partir d'un α,w-dihydrogénopolydiméthylsiloxane de masse moléculaire Mn = 22 800 g/mole (par dosage de SiH terminaux),
- sa Teneur pondérale en polydiméthylsiloxane est de : 90,0 %
- ses caractéristiques sont les suivantes : viscosité inhérente à 25 °C dans le chloroforme (concentration : 3 g/dl) : 0,39 dl/g.

On prépare une solution de 10,0 g de ce copolymère dans 90 g de toluène PROLABO-RECTAPUR®; on chauffe à 40 °C et on agite pendant 10 minutes pour faciliter la dissolution du copolymère

Ensuite, on charge 350 g de sphères de gel d'alginate de calcium dans un appareil d'enrobage par pulvérisation/séchage de type UNIGLATT® laboratoire, équipé d'un système de pulvérisa tion monté en "top-spray" (pulvérisation par le haut de l'appareil au-dessus du lit fluidisé).

Les sphères d'alginate sont caractérisées par un diamètre moyen de 4 mm et par une teneur en eau de 93 % minimum en poids. Ces sphères sont mises en suspension dans un flux d'air de fluidisation de 120 $m^3$/h à 30 °C.

La solution de copolymère dont la température est maintenue à 40 °C est pulvérisée dans le lit fluidisé dans les conditions suivantes :
- débit de solution de copolymère : 6,7 $cm^3$/mn,
- pression de l'air de pulvérisation : 1,2 bar,
- température de l'air de pulvérisation : 30 °C,
- durée de pulvérisation : 15 minutes

En fin d'opération, on recueille 228,6 g de sphères enrobées non collantes.

On a ensuite pris 100 g de ce produit et on l'a séché en étuve pendant 24 heures, à 60 °C, sous pression réduite (13,3 KPa) le séchage est terminé en présence de $P_2O_5$, jusqu'à obtenir un poids constant.

La teneur en eau du produit enrobé est de : 89,3 %.

L'analyse de surface met en évidence que les sphères d'alginate sont recouvertes d'un film continu et hydrophobe de copolymère thermoplastique.

EXEMPLE COMPARATIF 3 :

On répète exactement le mode opératoire de l'exemple 2, sauf qu'on utilise à la place du copolymère thermoplastique un polymère polydiméthylsiloxane bloqué à chaque extrémité de sa chaîne par un groupe triméthysilyle de masse moléculaire comprise entre 500 000 et 2 000 000 g/mole.

La pellicule formée présente des propriétés mécaniques insuffisantes et un caractère collant. En outre

les particules d'alginate enrobées s'agglomèrent entre elles pendant la pulvérisation et en cours de stockage.

EXEMPLE 4

ENROBAGE DES GRANULES DE KETOPROFENE

1. Synthèse et caractéristiques du copolymère organosiloxane utilisé

On a utilisé un copolymère organosiloxane préparé suivant le mode opératoire décrit à l'exemple 4 du brevet US-A-4 233 427.

L'α-w-dihydrogénopolydiméthylsiloxane utilisé, sur cette préparation a une masse moléculaire moyenne en nombre :

Mn = 22800 g/mole

(déterminée par dosage des fonctions SiH).

Ce copolymère est formé par une alternance de segments polydiméthylsiloxanes et de segments poly-(diméthylsilylène)phénylène (diméthylsilylène)-1,2-éthanediyl) répondant à la formule générale suivante :

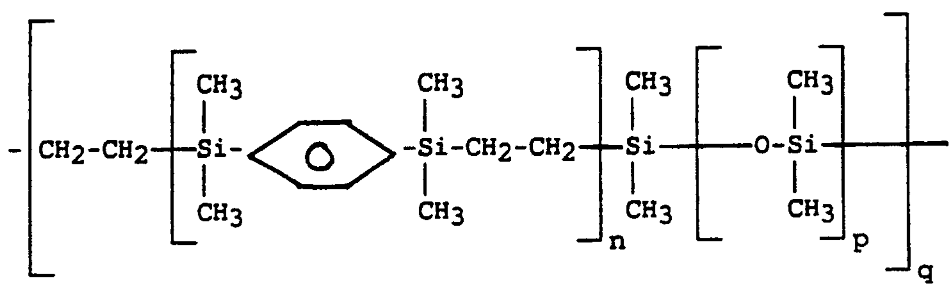

avec $\bar{n} \sim 11$ et $\bar{p} \sim 300$

La proportion de segments polydiméthylsiloxane dans le copolymère est de 90 (en poids).

La viscosité inhérente de ce copolymère est de 0,39 dl/g (mesurées à 25° C, sur une solution dans le chloroforme ayant une concentration de 3 g/dl).

2. Condition d'enrobaae

On utilise un appareil d'enrobage en lit fluidisé UNI-GLATT (R) équipé d'un système de pulvérisation WURSTER.

On prépare une solution du copolymère avec :

. 55,0 g de copolymère organosiloxane,

. 997 ml de dichloro-1,2-éthane.

On effectue deux opérations de pelliculage (A et B) en procédant selon le mode opératoire décrit ci-dessous. Chaque essai a été réalisé avec 350 g de granulés de kétoprofène obtenus de la manière suivante :

matières premières :

- Avicel PH 101, FMC (E.U.),
- Courlose, Courtaulds Acetate Ltd (G.B.),
- Kétoprofène B.P., RHONE-POULENC Ltd (G.B.)

Les granulés ont été préparés selon un procédé d'extrusion-sphéronisation. 3 kg de kétoprofène ont été mélangés avec 965,6 g d'Avicel PH 101 dans un mélangeur planétaire HOBART à la vitesse 1. Ce mélange de poudre a été granulé avec 2363 g d'une solution de Courlose à 1,5 % (p/p) pendant 2 minutes. La masse humide a été extrudée dans un appareil Fuji Paudal AXDCS-700, commercialisé par RUSSEL FINEX. Cet appareil était muni d'une grille percée de trous de 1 mm de diamètre et de 1 mm d'épaisseur.

Les corps extrudés cylindriques ont été introduits dans une machine à rouler des granulés sphériques (Spheronizer JB Caleva) pendant 3 mn et à une vitesse de 500 rpm. Ils ont ensuite été tamisés dans un appareil du type FRITSCH SIEVE ANALYSER pour que leur taille soit comprise entre 800 et 1400 µm.

On utilise les conditions de pelliculage suivantes :

. la température de la solution de copolymère est maintenue aux environs de 60° C pendant toute la durée de la pulvérisation,

. débit de la solution de copolymère : 6 ml/mn,

. pression de l'air de pulvérisation : 1,5 bar,

. température de l'air de pulvérisation : 40° C,

. les granulés sont brassés par un flux d'air de fluidisation de 75 $m^3$/h, ayant une température de 30° C.

Les autres caractéristiques de ces essais sont données sur le tableau suivant :

| N° D'ESSAI | VOLUME DE SOLUTION PULVERISEE (ML) | TAUX (1) D'ENROBANT CALCULE (%) | TITRE (2) EN KETOPROFENE (%) |
|---|---|---|---|
| A | 160 | 2,3 | 65 |
| B | 530 | 7,3 | 62 |

(1) en g de copolymère pour 100 g de granulés enrobés
(2) pondéral

CINETIQUE DE LIBERATION DU KETOPROFENE EN MILIEU TAMPONNE (pH=6.6) A 37° C

Description du test :

Dans un réacteur en verre de 1 litre muni d'un agitateur et chauffé par un bain d'eau thermostaté à 37° C, on charge 6 g de granulés et 1 litre de solution à pH = 6,6.

La solution tampon utilisée a été préparée de la manière suivante :

. dissolution de 68 g de dihydrogénophosphate de potassium ($KH_2PO_4$) dans 10 litres d'eau déminéralisée,

. ajustement du pH à 6,6 par coulée d'une solution de soude N.

Les courbes cinétiques ont été tracées à partir des dosages de kétoprofène réalisés à 260 nm, sur des prélèvements de la phase liquide, avec un spectrophotomètre UV/visible PHILIPS de type PYE UNICAM PU 8600.

La vitesse d'agitation de la suspension de granulés était de 300 rpm.

Les résultats de ces tests sont présentés sur la tableau I.

TABLEAU I

| TEMPS (h) | % DE KETOPROFENE LIBERE A pH = 6,6, 37 C | | |
|---|---|---|---|
| | NON ENROBE | A | B |
| 0,5 | 42,1 | | |
| 1 | 55,4 | 34,0 | 4,8 |
| 2 | 70,5 | 54,7 | 9,8 |
| 3 | 79,6 | | |
| 4 | 85,2 | 77,8 | 19,3 |
| 5 | | | 23,1 |
| 6 | | | 27,4 |
| 6,5 | 94,0 | | |
| 7 | 96,0 | | 31,1 |
| 8 | | 92,7 | 35,0 |
| 16 | | | 60,3 |
| 19,5 | | | 68,7 |
| 24 | | 100 | 81,0 |
| 26 | | | 84,2 |
| 28 | | | 87,3 |
| 32 | | | 92,6 |
| 48 | | | 100 |

EXEMPLE 5

On utilise dans cet exemple un copolymère linéaire multiséquencé thermoplastique préparé suivant la procédure décrite dans l'exemple 1 du brevet US-A-4 233 427.

L'α,w-dihydrogénopolydiméthylsiloxanne utilisé pour la synthèse de ce copolymère a une masse moléculaire moyenne en nombre, Mn, égale à 8600 g/mole (déterminée par dosage des groupements SiH terminaux).

Ce copolymère répond à la formule générale suivante :

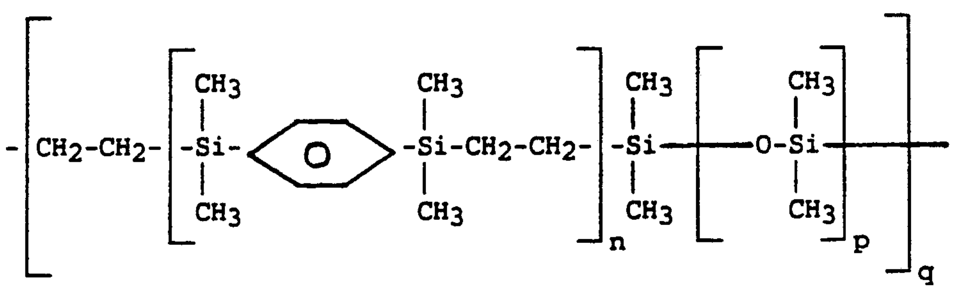

avec n ~ 13 et p ~ 115

La proportion de polydiméthylsiloxanne dans ce copolymère est de 75 % en poids.

La viscosité inhérente de ce copolymère est de 0,6 dl/g (mesuré à 25° C, sur une solution dans le chloroforme à 3 g de copolymère par dl).

Pelliculage

L'enrobage des grains de maïs est effectué par spray- coating dans un appareil AEROMATIC de type

AEROCOATER STREA-1 fonctionnant avec un système WURSTER.

Dans un récipient en verre, on prépare une solution de 7,5 g de copolymère dans 100 ml de toluène. Après avoir ajouté 2,25 g de MONTANE 85 (trioléate de sorbitan, de SEPIC), comme agent émulsifiant, on mélange à la solution 15 ml d'une dispersion aqueuse à 500 g/l de THIODICARB, un produit insecticide de RHONE-POULENC AGROCHIMIE.

L'addition de la dispersion aqueuse est effectuée à température ambiante, sous agitation avec un appareil POLYTRON tournant à 12000 tours/mn, pendant 2 à 3 minutes.

Ce mélange ainsi obtenu est pulvérisé à l'aide de l'appareil AEROMATIC que l'on a préalablement chargé avec 1,5 kg de graines de maïs.

Les conditions de pulvérisation sont les suivantes :
- débit d'air de fluidisation : 140 $m^3$/h
- température de l'air de fluidisation (sortie) : 30-34 °C
- pression de l'air de pulvérisation : 4 bars
- durée de pulvérisation : 17 minutes

Le dosage de THIODICARB montre que 81,8 % de la matière active insecticide pulvérisée se retrouve à la surface des graines de maïs, en fin d'opération.

Un test de germination de 9 jours, sur sable, à 20-25 °C, prenant comme échantillon témoin un lot de maïs nu non-traité, montre que le traitement n'est pas préjudiciable à la germination de la semence (90 % de germination pour le témoin nu, 96 % pour le produit pelliculé).

Par rapport à un maïs traité de façon classique au THIODICARB, sans copolymère, l'échantillon traité dans cet exemple présente une meilleure résistance à l'attrition, ainsi qu'une meilleure persistance de l'insecticide autour de la graine (grâce à l'effet de libération contrôlée apporté par le copolymère).

Ces avantages ont été mis en évidence par les tests suivants :

Résistance à l'attrition :

On place 50 g de semence dans un récipient en verre que l'on agite pendant 10 minutes, à l'aide d'un appareil de marque TURBULA tournant à 48 tours/minute - On pèse la quantité de fines qui se sont formées durant l'opération.

Résistance à l'élution par l'eau

On place 20 g de semence dans une colonne dans laquelle on fait couler 1 litre d'eau avec un débit de 12 ml/mn. A la fin du test, on détermine le pourcentage de matière active dans l'échantillon.

| . Résultat des tests : | | |
|---|---|---|
| | ATTRITION | ELUTION |
| Echantillon | de fines pour 100 g de maïs | THIODICARB résiduel, en % de la teneur initiale |
| Témoin maïs traité THIODICARB (traitement classique) | 0,417 | 57,3 |
| Echantillon de l'exemple, traité THIODICARB/COPOLYMERE | 0,085 | 98,5 |

**Revendications**

1. - Procédé d'encapsulation de particules de principe actif, de granulométrie comprise entre 50 µm et 5 mm par pelliculage de chaque particule au moyen d'un polymère, la pellicule étant obtenue par pulvérisation sur chaque particule d'un polymère en solution dans un solvant organique ou en émulsion ou dispersion aqueuses, suivie de l'évaporation du solvant et/ou de l'eau par séchage, caractérisé en ce que le polymère utilisé est un copolymère silicone thermoplastique.

2. - Procédé selon la revendication 1, caractérisé en ce que le copolymère silicone thermoplastique est

choisi parmi les copolymères multi-séquencés linéaires, les copolymères à blocs et les copolymères greffés dont la chaîne polymère principale sensiblement linéaire est constituée soit par une alternance de segments ou blocs polydiorganosiloxanes et de segments ou blocs organiques, soit par une chaîne polydiorganosiloxane sur laquelle sont greffés des chaînes organiques.

3. - Procédé selon la revendication 1 ou 2, caractérisé en ce que les séquences, blocs ou greffons du copolymère silicone thermoplastique sont choisis parmi des polyuréthannes, des polyarylènes, des polystyrènes, des polyesters, des polyéthers, des polycarbonates, des polyamides, des polyimides et des polyimides/amides.

4. - Procédé selon la revendication 3, caractérisé en ce que la teneur pondérale des séquences, blocs ou greffons organiques est comprise entre 5 et 60 %.

5. - Procédé selon l'une quelconque des revendications précédentes, caractérisé en ce que le copolymère silicone thermoplastique présente une température de transition vitreuse ou une température de fusion supérieure à 40 °C.

6.- Utilisation de copolymères silicones thermoplastiques pour l'encapsulation de principes actifs médicamenteux, phytosanitaires alimentaires

7.- Utilisation de copolymères selon l'une quelconque des revendications précédentes pour l'enrobage de principes actifs médicamenteux choisis parmi les agents antiinflammatoires, les hormones, les antibactériens, les agents antitumoraux.

8.- Utilisation de copolymères silicones thermoplastiques pour l'encapsulation de semences ou d'embryons végétaux.

9.- Principes actifs médicamenteux caractérisés en ce qu'ils sont enrobés d'un copolymère silicone thermoplastique.

10.- Ketoprofène caractérisé en ce qu'il est entouré d'un copolymère silicone thermoplastique.

11.- Principes actifs végétaux caractérisés en ce qu'ils sont enrobés d'un polymère silicone thermoplastique.

12.- Semences ou embryons végétaux caractérisés en ce qu'ils sont enrobés d'un polymère silicone thermoplastique.

Office européen des brevets

# RAPPORT DE RECHERCHE EUROPEENNE

Numero de la demande

EP 89 40 3546

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 281 204 (DOTT. BONAPACE & CO.)<br>--- | | B 01 J 13/04<br>A 61 K 9/50<br>A 01 N 25/24 |
| D,A | EP-A-0 280 400 (DOW CORNING CORP.)<br>--- | | |
| D,A | US-A-4 370 160 (M.J. ZIEMELIS)<br>--- | | |
| A | US-A-3 873 713 (W. HAAS et al.)<br>----- | | |
| | | | **DOMAINES TECHNIQUES RECHERCHES (Int. Cl.5)** |
| | | | B 01 J<br>A 61 K<br>A 01 N |

Le présent rapport a été établi pour toutes les revendications

| Lieu de la recherche | Date d'achèvement de la recherche | Examinateur |
|---|---|---|
| LA HAYE | 12-04-1990 | PYFFEROEN K. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant